# EUROPEAN PATENT SPECIFICATION

(11) **EP 2 935 461 B1**
(45) Date of publication and mention of the grant of the patent: **21.09.2016**
(21) Application number: 13818212.6
(22) Date of filing: 18.12.2013
(51) Int. Cl.: C08L 71/00, C08K 3/38, A61F 2/00

(54) **PROSTHETIC DEVICE**
PROTHESENVORRICHTUNG
DISPOSITIF DE PROTHÈSE

(30) Priority: 20.12.2012 US 201261740292 P; 12.02.2013 EP 13154978
(43) Date of publication of application: 28.10.2015
(73) Proprietor: Solvay Specialty Polymers USA, LLC., Alpharetta, GA 30005-3914 (US)
(72) Inventor: EL-HIBRI, Mohammad Jamal, Atlanta, Georgia 30350 (US); THOMAS, David B., Suwanee, Georgia 30024 (US)
(74) Representative: Benvenuti, Federica
(86) International application number: PCT/EP2013/077177
(87) International publication number: WO 2014/096058

(56) References cited:
- US-A1- 2009 163 955
- US-A1- 2010 241 166

## Description

This application claims priority to U.S. provisional application No. 61/740292 filed on 20 December 2012 and to European application No. 13154978.4 filed on 12 February 2013, the whole content of each of these applications being incorporated herein by reference for all purposes.

### Field of invention

The present invention is related to a prosthetic device comprising at least one structural part made of a poly(aryletherketone) polymer composition wherein said poly(aryletherketone) polymer composition is characterized by having improved mechanical properties, in particular having an excellent balance of stiffness and ductility, and thus are providing structural parts to be thinner and maintaining high stiffness.

### Background of the invention

Due both to demographic change and to developments in medical science, the number of surgical procedures involving prosthesis implantation is rising rapidly. The more obvious examples of prosthetic devices are hip or knee replacements and false teeth. Other less well-known examples are stents, heart valves, bone screws and plates and spinal fixators.

Prosthesis must be tolerated by the patient and not altered in time. Materials that may be suitable for each type of prosthesis are subjected to precise specifications. Indeed, if the prosthesis is a dental implant or a hip replacement the specifications will be very different.

The most important requirements are mechanical properties similar to those of bone to allow the transfer constraints between bone and prosthesis, chemical resistance to corrosion, chemical inertia in relation to the environment and biocompatibility. These properties must be controlled to maintain the integrity of used materials. The human body is an aggressive and corrosive environment mainly because of concentrations of chloride ions (113 mEq/l in blood plasma and 117 mEq/l in the interstitial fluid, which is sufficient to corrode metallic materials) and dissolved oxygen.

For dental implants, conditions are even tougher since the saliva contains more sulfur products that make it still more corrosive. The term "biocompatibility" is defined by the Dorland's Medical Dictionnary as the quality of not having toxic or injurious effects on biological systems. This encompasses both the material and host responses to an implant. The host response to an implant can be highly complex and is often linked to the material response. It is also dependent on the anatomical position of the implant. For a material to be biocompatible, it should not elicit any adverse host reactions to its presence. Inflammation and encapsulation phenomena may occur when the prosthesis suffer from low biocompatibility.

For some of the implants, as notably for dental implants, some challenging aesthetic requirements such as notably having very low discoloration effects of said implants also might be important.

Typically, prosthetic devices are made of inorganic (metal, alloys, ceramic and glass) and/or polymeric materials.

It should be noted that metals have the drawback that they are too stiff, and preventing the bone from bearing as much weight and causing the surrounding bone to disappear.

The use of polymeric materials for the manufacturing of prosthetic devices are for example described in U.S. 2007111165 whereby a prosthetic dental device is made of a thermoplastic polymer including a poly(aryl ketone), such as poly(ether ether ketone) (PEEK), polymethylmethacrylate (PMMA), poly(aryl ether ketone) (PAEK), poly(ether ketone) (PEK), poly(ether ketone ether ketone ketone) (PEKEKK), poly(ether ketone ketone) (PEKK), and/or polyetherimide (PEI), polysulfone (PSU), and polyphenylsulfone (PPSU).

The use of semi-crystalline polyaryletherketone (PAEK) polymers are for example also described in U.S. 4,662,887. Semi-crystalline (PAEK) polymers are suitable polymeric materials for use in the manufacturing of prosthetic devices, because said semi-crystalline polyaryletherketone (PAEK) polymers are known for their light weight, their exceptional balance of technical properties, namely high melting point, good thermal stability, high stiffness and strength, good toughness and really excellent chemical resistance (including environmental stress cracking resistance) and outstanding fatigue resistance, in addition to inertness to the body's environment.

As mentioned above, polymeric materials useful for providing structural prosthetic device parts should possess mechanical properties similar to those of bone. It is generally known that the stiffness of (PAEK) polymers can be increased by adding stiff materials such as reinforcing fillers, in particular glass fibers or carbon fibers however it has the drawback that said reinforced compositions often turn brittle.

Thus, there remains a continuous need for prosthetic devices comprising at least one part made of a polymeric composition that can overcome the drawbacks, mentioned above, and wherein said polymeric composition features excellent mechanical properties (and in particular good combination of high stiffness and high toughness, strength, elongation properties and impact resistance), having an excellent balance of stiffness and ductility, good processability, high chemical resistance, good biocompatibility and at the same time causing no discoloration or other degradation phenomena, and wherein said polymeric compositions provide thinner, lighter and stiffer parts, and the final parts and prosthetic devices have improved properties such as more uniform crystallinity, improved ductility, impact resistance, higher tensile and flex modulus as well as strength and moreover improved aesthetics, especially an improved, lighter color.

### Brief description of the figures

Illustrated in sectional view in the drawings are, in Fig. 1, a cranial plate 1 in situ within an aperture cut into the skull 2 ; in Fig. 2 an insert 3 in a long bone 4 from which part of the bone had been removed ; and in Fig. 3 a femur head replacement 5 in situ within a femur 6.

This last device comprises a core component 7 of a cement composition as described in European Patent Specification No. 21682 and comprising a core component 7 and/or a surface coating 8 of the polymer composition (C) a bearing surface 9 of titanium and a fibrous surface layer 10.

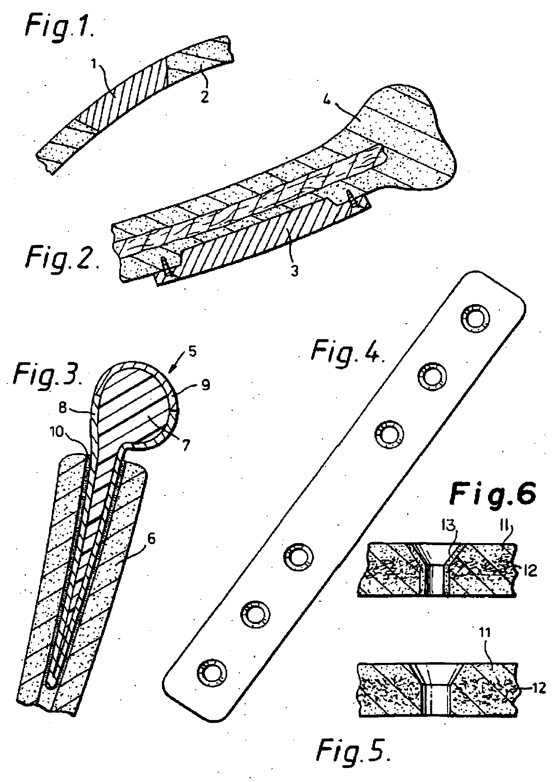

A bone plate shown in perspective in Fig. 4 is shown also in enlarged section in Fig. 5, to illustrate the use of the polymer composition (C) outer coating 11 upon a core region comprising a composite of carbon fibre with the polymer composition (C). Fig. 6 shows the presence of the polymer composition (C) 13 also after refilling of the screw holes and redrilling.

### Summary of invention

The present invention addresses the above detailed needs and relates to a prosthetic device comprising at least one part made of a polymer composition [composition (C), herein after] comprising
(i) at least one polyaryletherketone polymer [(PAEK) polymer],
(ii) at least one nitride (NI) of an element having an electronegativity (ε) of from 1.3 to 2.5, as defined in « Handbook of Chemistry and Physics », CRC Press, 64th edition, pages B-65 to B-158.

### The prosthetic device

To the purposes of the invention, the term "prosthetic device" is intended to denote an artificial device which is made to replace and act as a missing biological structure. Prosthetic devices may have structural features which make them suitable to act as reinforcement or replacement of a missing or defective animal or human body part, e.g. a bone implant. Prosthetic devices of many shapes, configurations and properties are commonly employed within the living body. They can be used to replace parts lost by injury (traumatic or chirurgical) or missing from birth (congenital) or to supplement defective body parts.

For the sake of clarity, the term "part of a prosthetic device" is intended to denote a piece or portion which is combined with others to make up the whole prosthetic device. The external coating of a prosthetic device falls thus within this scope.

The prosthetic device of the present invention may comprise additional parts. Additional parts are intended to denote parts of the prosthetic devices which do not aim to replace a part of the body as such, but perform a supplementary function. For instance, it may comprise metal inserts, structural reinforcements, radio-opaque inserts, moving motor-driven assemblies, electronic devices, controlling units and the like.

The prosthetic device according to the present invention may be an orthopaedic prosthesis for building and/or repairing and/or improving surface properties of skeletal bones and joints such as, but not limited to ligaments, tendons, cartilage, bones, hip joints, knee prosthesis, spinal disc orthoprosthesis.

Orthopaedic prostheses comprise manufactured replacements for the ends and articulating surfaces of the bones of the skeleton. Such prostheses are generally implanted to repair or reconstruct all or part of an articulating skeletal joint that is functioning abnormally due to disease, trauma or congenital defect. Other forms of implantable orthopaedic prostheses, beyond providing manufactured replacements for the ends and articulating surfaces of the bones of the skeletal joints, also provide manufactured replacements for portions of the bones distant from the articulating surface. These other forms may be used in cases of abnormally extensive atrophy or resorption of bone in the vicinity of the articulating surface or prior implant, or in cases where an extensive amount of bone is to be intentionally resected to treat oncological or other diseases of the bone. Because the natural bony areas to which ligaments, tendons and other soft tissues attach are often lost to such extensive resections of the bone, implantable orthopaedic implants designed for such cases often include means for attaching bone and/or soft tissue directly to the implant. Generally such means also provide an initial mechanical attachment, supplemented by later ingrowth and ongrowth of the bone and soft tissue to the prosthesis.

For example, the prosthetic device of the present invention may be selected from the group consisting of :
- orthopaedic prosthesis such as ligaments, tendons, cartilage, bones, hip joints, knee prosthesis, spinal disc orthoprosthesis ;
- dental structures such as dentures, partial dentures ;
- prosthetic structures for other body parts, such as prosthetic devices that serve as artificial body parts including limbs, eyes, implants, included cosmetic implants, hearing aids, and the like, such as spectacle frames ;
- fixed prosthetic anatomical devices such as caps, crowns and other non-dental anatomical replacement structures.

### The polyaryletherketone polymer

Within the context of the present invention the mention "at least one polyaryletherketone polymer [(PAEK) polymer]" is intended to denote one or more than one (PAEK) polymer. Mixtures of (PAEK) polymer can be advantageously used for the purposes of the invention.

In the rest of the text, the expressions "(PAEK) polymer" are understood, for the purposes of the present invention, both in the plural and the singular, that is to say that the inventive composition may comprise one or more than one (PAEK) polymer.

For the purpose of the invention, the term "polyaryletherketone (PAEK)" is intended to denote any polymer, comprising recurring units, more than 50 % moles of said recurring units are recurring units (R_{PAEK}) comprising a Ar-C(O)-Ar' group, with Ar and Ar', equal to or different from each other, being aromatic groups. The recurring units (R_{PAEK}) are generally selected from the group consisting of formulae (J-A) to (J-O), herein below : 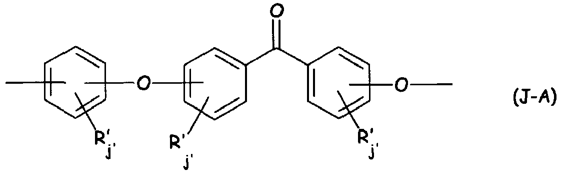 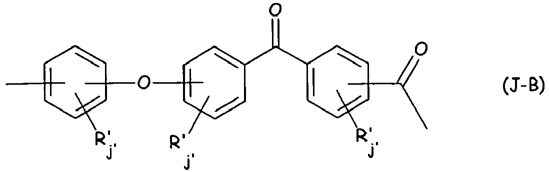 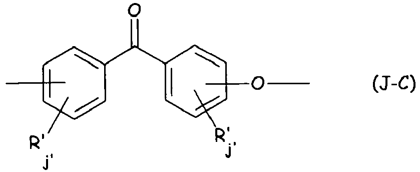 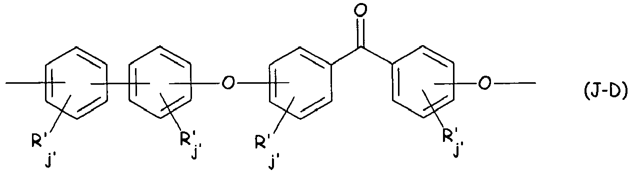 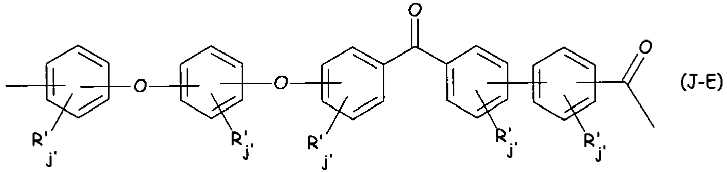 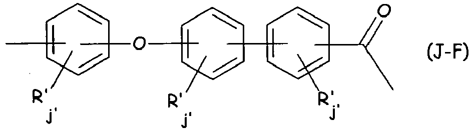 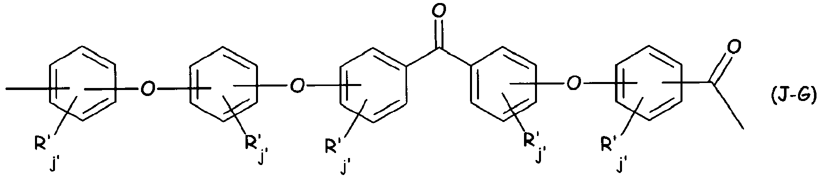 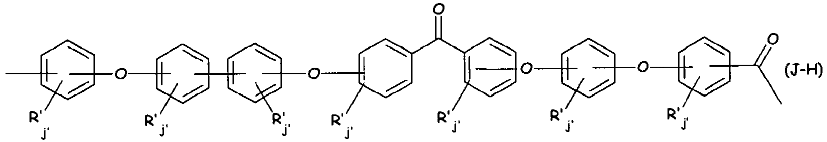 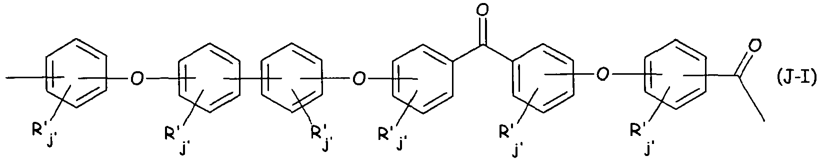 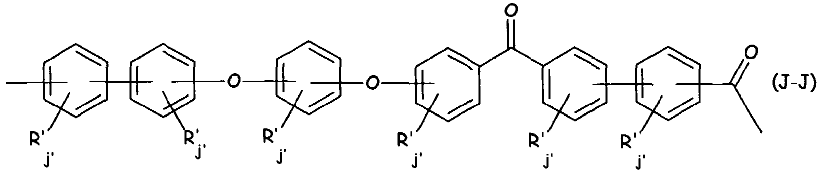 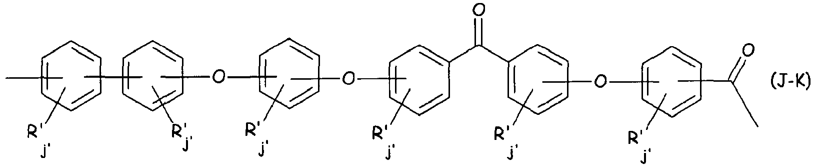 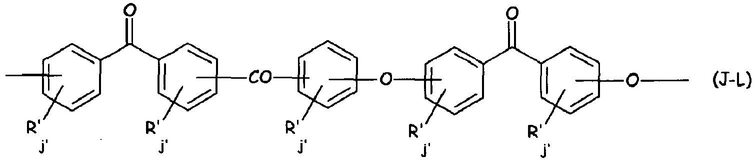 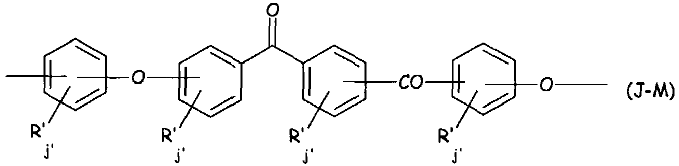 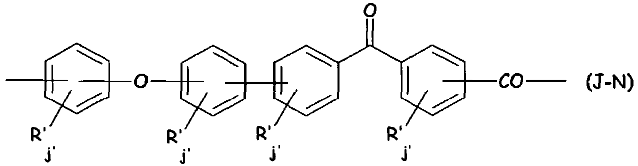 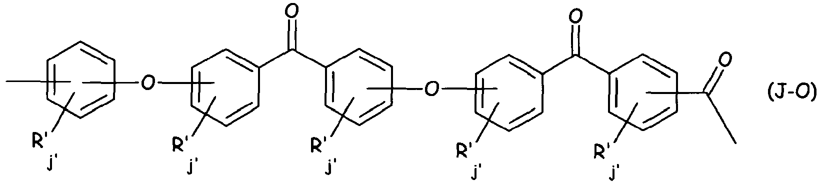 wherein :
- each of R', equal to or different from each other, is selected from the group consisting of halogen, alkyl, alkenyl, alkynyl, aryl, ether, thioether, carboxylic acid, ester, amide, imide, alkali or alkaline earth metal sulfonate, alkyl sulfonate, alkali or alkaline earth metal phosphonate, alkyl phosphonate, amine and quaternary ammonium ;
- j' is zero or is an integer from 0 to 4.

In recurring unit (R_{PAEK}), the respective phenylene moieties may independently have 1,2-, 1,4- or 1,3 -linkages to the other moieties different from R' in the recurring unit. Preferably, said phenylene moieties have 1,3-or 1,4- linkages, more preferably they have 1,4-linkage.

Still, in recurring units (R_{PAEK}), j' is at each occurrence zero, that is to say that the phenylene moieties have no other substituents than those enabling linkage in the main chain of the polymer.

Preferred recurring units (R_{PAEK}) are thus selected from those of formulae (J'-A) to (J'-O) herein below :

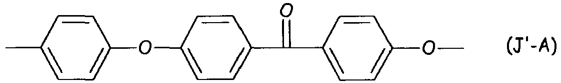

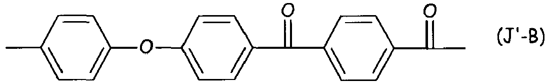

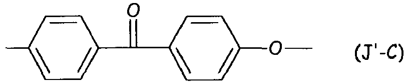

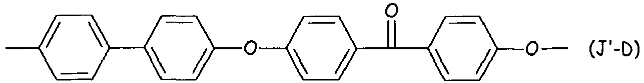

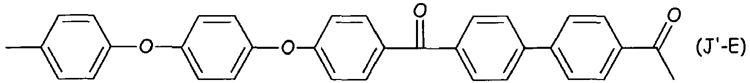

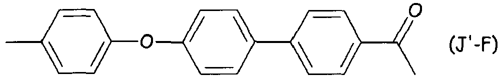

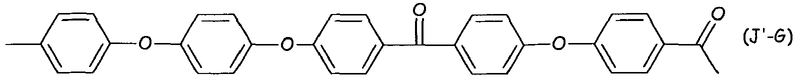

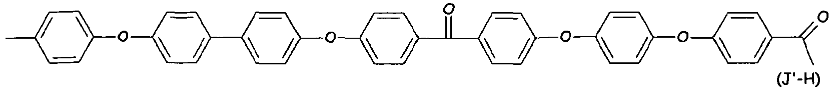

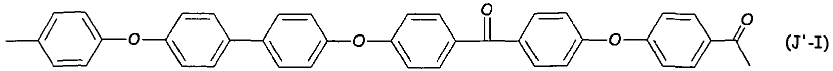

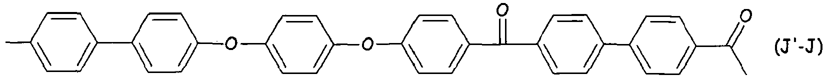

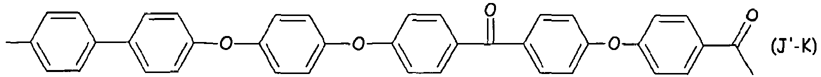

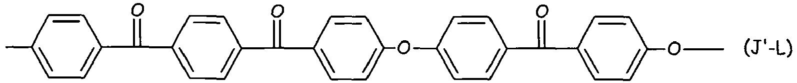

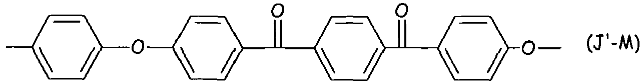

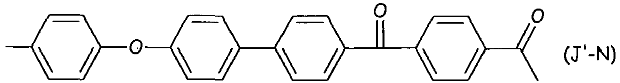

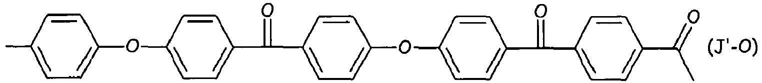

In the (PAEK) polymer, as detailed above, preferably more than 60 %, more preferably more than 80 %, still more preferably more than 90 % moles of the recurring units are recurring units (R_{PAEK}), as above detailed.

Still, it is generally preferred that substantially all recurring units of the (PAEK) polymer are recurring units (R_{PAEK}), as detailed above ; chain defects, or very minor amounts of other units might be present, being understood that these latter do not substantially modify the properties of (R_{PAEK}).

The (PAEK) polymer may be notably a homopolymer, a random, alternate or block copolymer. When the (PAEK) polymer is a copolymer, it may notably contain (i) recurring units (R_{PAEK}) of at least two different formulae chosen from formulae (J-A) to (J-O), or (ii) recurring units (R_{PAEK}) of one or more formulae (J-A) to (J-O) and recurring units (R*_{PAEK}) different from recurring units (R_{PAEK}).

As will be detailed later on, the (PAEK) polymer may be a polyetheretherketone polymer [(PEEK) polymers, herein after]. Alternatively, the (PAEK) polymer may be a polyetherketoneketone polymer [(PEKK) polymer, herein after], a polyetherketone polymer [(PEK) polymer, hereinafter], a polyetheretherketoneketone polymer [(PEEKK) polymer, herein after], or a polyetherketoneetherketoneketone polymer [(PEKEKK) polymer, herein after].

The (PAEK) polymer may also be a blend composed of at least two different (PAEK) polymers chosen from the group consisting of (PEKK) polymers, (PEEK) polymers, (PEK) polymers and (PEKEKK) polymers, as above detailed.

For the purpose of the present invention, the term "(PEEK) polymer" is intended to denote any polymer of which more than 50 % by moles of the recurring units are recurring units (R_{PAEK}) of formula J'-A.

Preferably more than 75 % by moles, preferably more than 85 % by moles, preferably more than 95 % by moles, preferably more than 99 % by moles of the recurring units of the (PEEK) polymer are recurring units of formula J'-A. Most preferably all the recurring units of the (PEEK) polymer are recurring units of formula J'-A.

For the purpose of the present invention, the term "(PEKK) polymer" is intended to denote any polymer of which more than 50 % by moles of the recurring units are recurring units (R_{PAEK}) of formula J'-B.

Preferably more than 75 % by moles, preferably more than 85 % by moles, preferably more than 95 % by moles, preferably more than 99 % by moles of the recurring units of the (PEKK) polymer are recurring units of formula J'-B. Most preferably all the recurring units of the (PEKK) polymer are recurring units of formula J'-B.

For the purpose of the present invention, the term "(PEK) polymer" is intended to denote any polymer of which more than 50 % by moles of the recurring units are recurring units (R_{PAEK}) of formula J'-C.

Preferably more than 75 % by moles, preferably more than 85 % by moles, preferably more than 95 % by moles, preferably more than 99 % by moles of the recurring units of the (PEK) polymer are recurring units of formula J'-C. Most preferably all the recurring units of the (PEK) polymer are recurring units of formula J'-C.

For the purpose of the present invention, the term "(PEEKK) polymer" is intended to denote any polymer of which more than 50 % by moles of the recurring units are recurring units (R_{PAEK}) of formula J'-M.

Preferably more than 75 % by moles, preferably more than 85 % by moles, preferably more than 95 % by moles, preferably more than 99 % by moles of the recurring units of the (PEEKK) polymer are recurring units of formula J'-M. Most preferably all the recurring units of the (PEEKK) polymer are recurring units of formula J'-M.

For the purpose of the present invention, the term "(PEKEKK) polymer" is intended to denote any polymer of which more than 50 % by moles of the recurring units are recurring units (R_{PAEK}) of formula J'-L.

Preferably more than 75 % by moles, preferably more than 85 % by moles, preferably more than 95 % by moles, preferably more than 99 % by moles of the recurring units of the (PEKEKK) polymer are recurring units of formula J'-L. Most preferably all the recurring units of the (PEKEKK) polymer are recurring units of formula J'-L.

Excellent results were obtained when the (PAEK) polymer was a (PEEK) homopolymer, i.e. a polymer of which substantially all the recurring units of the (PEEK) polymer are recurring units of formula J'-A, wherein chain defects, or very minor amounts of other units might be present, being understood that these latter do not substantially modify the properties of the (PEEK) homopolymer.

Non limitative examples of commercially available polyaryletherketone (PAEK) resins suitable for the invention include the KETASPIRE^{®} polyetheretherketone commercially available from Solvay Specialty Polymers USA, LLC.

The (PAEK) polymer can have a intrinsic viscosity (IV) of at least 0.50 dl/g, preferably at least 0.60 dl/g, more preferably at least 0.70 dl/g, as measured in 95-98 % sulfuric acid (d= 1.84 g/ml) at a (PAEK) polymer concentration of 0.1 g/100 ml.

The IV of the (PAEK) polymer can notably be equal to or less than 1.40 dl/g, preferably equal to or less than 1.30 dl/g, more preferably equal to or less than 1.20 dl/g, most preferably equal to or less than 1.15 dl/g, as measured in 95-98 % sulfuric acid (d= 1.84 g/ml) at a (PAEK) polymer concentration of 0.1 g/100 ml.

Good results have been obtained with (PAEK) polymers having an IV from 0.70 dl/g to 1.15 dl/g, as measured in 95-98 % sulfuric acid (d= 1.84 g/ml) at a (PAEK) polymer concentration of 0.1 g/100 ml.

The measurement is generally performed using a No 50 Cannon-Fleske viscometer ; IV is measured at 25°C in a time less than 4 hours after dissolution.

The (PAEK) polymer has a melt viscosity of advantageously at least 0.05 kPa.s, preferably at least 0.08 kPa.s, more preferably at least 0.1 kPa.s, still more preferably at least 0.12 kPa.s at 400°C and a shear rate of 1000 s⁻¹, as measured using a capillary rheometer in accordance with ASTM D3835.

As capillary rheometer, a Kayeness Galaxy V Rheometer (Model 8052 DM) can be used.

The PAEK polymer has a melt viscosity of advantageously at most 1.00 kPa.s, preferably at most 0.80 kPa.s, more preferably at most 0.70 kPa.s, even more preferably at most 0.60 kPa.s, most preferably at most 0.50 kPa.s at 400°C and a shear rate of 1000 s⁻¹, as measured using a capillary rheometer in accordance with ASTM D3835.

The (PAEK) polymer can be prepared by any method known in the art for the manufacture of poly(aryl ether ketone)s.

### The nitride (NI)

Within the context of the present invention the mention "at least one nitride (NI)" is intended to denote one or more than one nitride (NI). Mixtures of nitrides (NI) can be advantageously used for the purposes of the invention.

For the purpose of the present invention, an "element" is intended to denote an element from the Periodic Table of the Elements.

The value of the electronegativity of an element that are to be taken into consideration for the purpose of the present invention are those reported in the Periodic Table of the Elements edited by J. Breysem, c/o VEL s.a., "Produits, appareillage et fournitures pour le laboratoire", printed in Belgium in February 1987.

Non limitative examples of nitrides (NI) of an element having an electronegativity (ε) of from 1.3 to 2.5 are listed « Handbook of Chemistry and Physics », CRC Press, 64^{th} edition, pages B-65 to B-158. The code into brackets is the one attributed by the CRC Handbook to the concerned nitride, while ε denotes the electronegativity of the element from which the nitride is derived. Then, nitrides (NI) of an element having an electronegativity (ε) of from 1.3 to 2.5 suitable to the purpose of the present invention are notably aluminum nitride (AlN, a45, ε=1.5), antimony nitride (SbN, a271, ε=1.9), beryllium nitride (Be₃N₂, b123, ε=1.5), boron nitride (BN, b203, ε=2.0), chromium nitride (CrN, c406, ε=1.6), copper nitride (Cu₃N, c615, ε=1.9), gallium nitride (GaN, g41, ε=1.6), trigermanium dinitride (Ge₃N₂, g82, ε=1.8), trigermanium tetranitride (Ge₃N₄, g83, ε=1.8), hafnium nitride (HfN, h7, ε=1.3), iron nitrides like Fe₄N (i151, ε=1.8) and Fe₂N or Fe₄N₂ (i152, ε=1.8), mercury nitride (Hg₃N₂, m221, ε=1.9), niobium nitride (n109, ε=1.6), silicium nitride (Si₃N₄, s109, ε=1.8), tantalum nitride (TaN, t7, ε=1.5), titanium nitride (Ti₃N₄, t249, ε=1.5), wolfram dinitride (WN₂, t278, ε=1.7), vanadium nitride (VN, v15, ε=1.6), zinc nitride (Zn₃N₂, z50, ε=1.6) and zirconium nitride (ZrN, z105, ε=1.4).

The nitride (NI) is a nitride of an element having an electronegativity of preferably at least 1.6, and more preferably at least 1.8. In addition, the nitride (NI) is the nitride of an element having an electronegativity of preferably at most 2.2.

Besides, the nitride (NI) is chosen preferably from nitrides of an element chosen from Groups IIIa, IVa, IVb, Va, Vb, VIa, VIb, VIIb and VIII of the Periodic Table of the Elements, and more preferably from nitrides of an element of Group IIIa of the Periodic Table of the Elements.

The most preferred nitride (NI) is boron nitride.

The Applicant has surprisingly found that the presence of the nitride (NI), as described above, is effective in enhancing the stiffness of the composition (C) while maintaining the ductility of the unmodified PAEK polymer used thus far, thereby offering said composition (C) of the invention superior properties which allows them to be very useful as being comprised in parts of the prosthetic devices.

The Applicant has found that the average particle size of the nitride (NI) may play a role in improving mechanical properties such as in particular the stiffness and the tensile elongation at break of the composition (C) and in improving the aesthetics aspects, especially in improved the color of the composition (C).

The average particle size of the nitride (NI) is advantageously equal to or below 30 µm, preferably equal to or below 20 µm, more preferably equal to or below 18 µm, more preferably equal to or below 10 µm.

The average particle size of the nitride (NI) is preferably equal to or at least 0.05 µm, equal to or at least 0.1 µm, more preferably equal to or at least 0.2 µm, equal to or at least 1 µm.

The average particle size of the nitride (NI) is preferably from 1 µm to 20 µm, more preferably from 2 µm to 18 µm, more preferably from 2 µm to 10 µm.

An average particle size of the nitride (NI) of about 2.5 µm gave particularly good results.

The average particle size of the nitride (NI) is measured via light scattering techniques (dynamic or laser) using the respective equipment coming for example from the company Malvern (Mastersizer Micro or 3000) or using screen analysis according to DIN 53196.

### Composition (C)

The composition (C) of the present invention advantageously comprises the nitride (NI) in an amount of at least 1.0 % wt, preferably at least 1.10 % wt, more preferably at least 2.0 % wt, most preferably at least 5.0 % wt based on the total weight of the composition (C).

As such, there is no upper limit on the amount of the nitride (NI) present in the composition (C) of the present invention.

In one embodiment, the composition (C) of the present invention advantageously comprises the nitride (NI) in an amount of at most 50.0 % wt, preferably at most 40.0 % wt, more preferably at most 30.0 % wt, even more preferably at most 20.0 % wt, still more preferably at most 15.0 % wt, and most preferably at most 10.0 % wt, based on the total weight of the composition (C).

The composition (C) of the present invention advantageously comprises the nitride (NI) in an amount ranging from 2 to 50 % wt, more preferably from 5 to 20 % wt, even more preferably from 5 to 10 % wt, based on the total weight of the composition (C).

The total weight of the the (PAEK) polymer, based on the total weight of the composition (C), is advantageously above 50 %, preferably above 60 % ; more preferably above 70 % ; more preferably above 80 %, more preferably above 85 %.

If desired, the composition (C) consists of the (PAEK) polymer and the nitride (NI).

A preferred composition (C) of the invention thus includes a (PAEK) polymer, as above detailed, and more preferably a (PAEK) polymer comprising recurring units (R_{PAEK}) of formula (J'-A), as above detailed and boron nitride in an amount of 5 to 15 % wt, based on the total weight of the composition (C).

The composition (C) of the present invention may further comprise at least one other thermoplastic polymer (polymer T).

Non limitative examples of polymers (T) suitable for use in composition (C) of the present invention, include for example polyarylethersulfones, polyphenylenes, polyimides, more notably polyetherimides, and polyphenylene sulfides.

The weight of said other polymers is advantageously below 40 % wt, preferably below 30 % wt, and more preferably below 25 % wt based on the total weight of the composition (C).

The composition (C) can further comprise one or more ingredients other than the (PAEK) polymer [ingredient (I), herein after].

Non limitative examples of ingredient (I) suitable for use in composition (C) of the present invention, are polymeric compositions, additives such as UV absorbers ; stabilizers such as light stabilizers and heat stabilizers ; antioxidants ; lubricants ; processing aids ; plasticizers ; flow modifiers ; flame retardants ; pigments such as notably titanium dioxide (TiO₂) ; dyes ; colorants ; anti-static agents ; extenders ; metal deactivators ; conductivity additive such as carbon black and carbon nanofibrils and combinations comprising one or more of the foregoing additives.

The weight of said ingredient (I) is advantageously below 10 % wt and preferably below 5 % wt, based on the total weight of the composition (C).

If desired, the composition (C) comprises more than 80 wt % of the (PAEK) polymer with the proviso that the (PAEK) polymer is the only polymeric components in the composition (C) and one or more optional ingredient such as notably UV absorbers ; stabilizers such as light stabilizers and heat stabilizers ; antioxidants ; lubricants ; processing aids ; plasticizers ; flow modifiers ; flame retardants ; pigments such as notably titanium dioxide (TiO₂) ; dyes ; colorants ; anti-static agents ; extenders ; metal deactivators ; conductivity additive such as carbon black and carbon nanofibrils might be present therein, without these components dramatically affecting relevant mechanical and toughness properties of the composition (C).

The expression 'polymeric components' is to be understood according to its usual meaning, i.e. encompassing compounds characterized by repeated linked units, having typically a molecular weight of 2 000 or more.

The polymer composition (C) may further comprise at least one reinforcing filler. Reinforcing fillers are well known by the skilled in the art. They are preferably selected from fibrous and particulate fillers different from the pigment as defined above. More preferably, the reinforcing filler is selected from mineral fillers (such as talc, mica, kaolin, calcium carbonate, calcium silicate, magnesium carbonate), glass fiber, carbon fibers, synthetic polymeric fiber, aramid fiber, aluminum fiber, titanium fiber, magnesium fiber, boron carbide fibers, rock wool fiber, steel fiber, wollastonite etc. Still more preferably, it is selected from mica, kaolin, calcium silicate, magnesium carbonate, glass fiber, carbon fibers and wollastonite etc.

Preferably, the filler is chosen from fibrous fillers. A particular class of fibrous fillers consists of whiskers, i.e. single crystal fibers made from various raw materials, such as Al₂O₃, SiC, BC, Fe and Ni.

In one embodiment of the present invention the reinforcing filler is chosen from wollastonite and glass fiber. Among fibrous fillers, glass fibers are preferred ; they include chopped strand A-, E-, C-, D-, S-, T- and R-glass fibers, as described in chapter 5.2.3, p. 43-48 of Additives for Plastics Handbook, 2^{nd} edition, John Murphy.

Glass fibers optionally comprised in polymer composition (C) may have a circular cross-section or a non-circular cross-section (such as an oval or rectangular cross-section).

When the glass fibers used have a circular cross-section, they preferably have an average glass fiber diameter of 3 to 30 µm and particularly preferred of 5 to 12 µm. Different sorts of glass fibers with a circular cross-section are available on the market depending on the type of the glass they are made of. One may notably cite glass fibers made from E- or S-glass.

Good results were obtained with standard E-glass material with a non-circular cross section. Excellent results were obtained when the polymer composition with S-glass fibers with a round cross-section and, in particular, when using round cross-section with a 6 µm diameter (E-Glass or S-glass).

In another embodiment of the present invention the reinforcing filler is a carbon fiber.

As used herein, the term "carbon fiber" is intended to include graphitized, partially graphitized and ungraphitized carbon reinforcing fibers or a mixture thereof. Carbon fibers useful for the present invention can advantageously be obtained by heat treatment and pyrolysis of different polymer precursors such as, for example, rayon, polyacrylonitrile (PAN), aromatic polyamide or phenolic resin ; carbon fibers useful for the present invention may also be obtained from pitchy materials. The term "graphite fiber" intends to denote carbon fibers obtained by high temperature pyrolysis (over 2000°C) of carbon fibers, wherein the carbon atoms place in a way similar to the graphite structure. Carbon fibers useful for the present invention are preferably chosen from the group composed of PAN-based carbon fibers, pitch based carbon fibers, graphite fibers, and mixtures thereof.

The weight of said reinforcing filler is advantageously preferably below 60 % wt, more preferably below 50 % wt, even more preferably below 45 % wt, most preferably below 35 % wt, based on the total weight of the composition (C).

Preferably, the reinforcing filler is present in an amount ranging from 10 to 60 % wt, preferably from 20 to 50 % wt, preferably from 25 to 45 % wt, most preferably from 25 to 35 % wt, based on the total weight of the polymer composition (C).

The composition (C) can be prepared by a variety of methods involving intimate admixing of the polymer materials with any optional ingredient, as detailed above, desired in the formulation, for example by melt mixing or a combination of dry blending and melt mixing. Typically, the dry blending of the (PAEK) polymer and the nitride (NI), and optionally the polymers (T), optionally the reinforcing filler and optionally ingredient (I), as above details, is carried out by using high intensity mixers, such as notably Henschel-type mixers and ribbon mixers.

So obtained powder mixture can comprise the (PAEK) polymer and the nitride (NI), and optionally the polymers (T), optionally the reinforcing filler and optionally ingredient (I), in the weight ratios as above detailed, suitable for obtaining effective formation of the above described parts of a prosthetic device, or can be a concentrated mixture to be used as masterbatch and diluted in further amounts of the (PAEK) polymer and the nitride (NI), and optionally the polymers (T), optionally the reinforcing filler and optionally ingredient (I) in subsequent processing steps.

It is also possible to manufacture the composition of the invention by further melt compounding the powder mixture as above described. As said, melt compounding can be effected on the powder mixture as above detailed, or preferably directly on the (PAEK) polymer and the nitride (NI), and optionally the polymers (T), optionally the reinforcing filler and optionally ingredient (I). Conventional melt compounding devices, such as co-rotating and counterrotating extruders, single screw extruders, co-kneaders, disc-pack processors and various other types of extrusion equipment can be used. Preferably, extruders, more preferably twin screw extruders can be used.

If desired, the design of the compounding screw, e.g. flight pitch and width, clearance, length as well as operating conditions will be advantageously chosen so that sufficient heat and mechanical energy is provided to advantageously fully melt the powder mixture or the ingredients as above detailed and advantageously obtain a homogeneous distribution of the different ingredients. Provided that optimum mixing is achieved between the bulk polymer and filler contents. It is advantageously possible to obtain strand extrudates which are not ductile of the composition (C) of the invention. Such strand extrudates can be chopped by means e.g. of a rotating cutting knife after some cooling time on a conveyer with water spray. Thus, for example composition (C) which may be present in the form of pellets or beads can then be further used for the manufacture of the above described part of a prosthetic device.

Another objective of the present invention is to provide a method for the manufacture of the above described part of a prosthetic device device. Such method is not specifically limited. The polymer composition (C) may be generally processed by injection molding, extrusion or other shaping technologies.

In one embodiment of the present invention, the method for the manufacture of the above described part of a mobile electronic device includes the step of injection molding and solidification of the polymer composition (C).

In another embodiment of the present invention, the method for the manufacture of the above described part of a prosthetic device includes the machining of a standard shaped structural part in a part having any type of size and shape. Non limiting examples of said standard shaped structural part include notably a plate, a rod, a slab and the like. Said standard shaped structural parts can be obtained by extrusion or injection molding of the polymer composition (C).

Another object of the invention is a part of a prosthetic device comprising the polymer composition as above described.

The Applicant has found unexpectedly that the composition (C) of the present invention is effective in providing prosthetic device parts having higher stiffness than devices of the prior art while at the same time not sacrificing the ductility, elongation, toughness and impact resistance properties of the unmodified PAEK resin that has been used thus far. Impact resistance is particularly important in prosthetic applications because in many cases the surgeon has to hammer the part into position.

The Applicant has also found that said prosthetic device parts comprising the composition (C) of the present invention have improved aesthetics, in particular improved lighter color and said prosthetic device parts have a higher acceptance for many applications where color is a concern, as notably in dental applications, and other cosmetic applications.

The prosthetic device parts of the present invention have advantageously the following color characteristics :
- Color L* > 70, preferably L* > 71 ;
- Color b* is at least 8
where the color was measured on injection moulded color plaques that are 2.5 mm in thickness using the CIE Lab standards, as follows. The color is generally characterized by L*, a*, b* values, which are tristimulus coordinates defined by the CIE (Commission Internationale de l'Eclairage) in 1976 (K. Nassau, in "Kirk-Othmer Encylopedia of Chemical Technology", 2004, Chapter 7, P 303-341). These three basic coordinates represent the lightness of the color (L*, L* = 0 yields black and L* = 100 indicates white), its position between red/magenta and green (a*, negative values indicate green while positive values indicate magenta) and its position between yellow and blue (b*, negative values indicate blue and positive values indicate yellow).

Should the disclosure of any patents, patent applications, and publications which are incorporated herein by reference conflict with the description of the present application to the extent that it may render a term unclear, the present description shall take precedence.

The invention will be now described in more details with reference to the following examples, whose purpose is merely illustrative and not intended to limit the scope of the inventior.

### Raw materials

KetaSpire^{®} PEEK KT-820P is polyetheretherketone polymer commercially available from Solvay Specialty Polymers USA, LLC.

Boron Nitride, Boronid^{®} S1-SF commercially available from ESK Ceramics, GmbH, average particle size of 2.5 µm.

Boron Nitride, Boronid^{®} S15 commercially available from ESK Ceramics, GmbH, average particle size of 15 µm.

Carbon Fiber, Sigrafil C30 APS 006 from SGL Corporation Talc, Mistron Vapor R, commercially available from Luzenac America

### General description of the compounding process of PEEK resins

A dry blend of PEEK resins with the desired amounts of Boronid^{®} S1-SF or Boronid^{®} S15 were prepared by first tumble blending for about 20 minutes, followed by melt compounding using an 25 mm Berstorff co-rotating partially intermeshing twin screw extruder having an L/D ratio of 40:1. The extruder had 8 barrel sections with barrel sections 2 through 8 being heated sections. Vacuum venting was applied at barrel section 7 with 18-20 in of vacuum during compounding to strip off moisture and any possible residual volatiles from the compound. The compounding temperature profile was such that barrel sections 2-5 were set at 330°C while barrel sections 5-8 and the die adapter were set at 340°C. The screw speed used 180 throughout and the throughput rate was 15-17 lb/hr, whereas the melt temperature, measured manually for each formulation molten extrudate, at the exit of the extruder die ranged from 398 to 402°C. The extrudate for each formulation was cooled in a water trough and then pelletized using a pelletizer. The thus obtained pellets of the four blends were next dried for 4 hours in a desiccated air oven at 150°C and subjected to mechanical testing. Said pellets were injection-molded to produce ASTM test specimens using a Toshiba 150 ton injection molding machine following standard conditions and guidelines for KetaSpire KT-820 PEEK resin provided by the supplier Solvay Specialty Polymers.

Mechanical properties were tested for all the formulations using injection molded 0.125 inch thick ASTM test specimens which consisted of 1) Type I tensile bars, 2) 5 in x 0.5 in x 0.125 in flexural bars, and 3) 4 in x 4 in x 0.125 in plaques for the instrumented impact (Dynatup) testing.

The following ASTM test methods were employed in evaluating all nine compositions :
- D638 : Tensile properties using a test speed of 2 in/min
- D790 : Flexural properties
- D256 : Izod impact resistance (notched)
- D4812 : Izod impact resistance (unnotched)
- D3763 : Instrumented impact resistance also known by the name Dynatup impact
- D648 : Heat deflection temperature (HDT)
- D5279 : DMA Storage Modulus at 200°C (Pa)

HDT was measured at an applied stress of 264 psi and using 0.125 in-thick flexural specimens annealed at 200°C for 2 hours to assure uniform crystallinity and removal of residual molded-in stresses in the parts which can otherwise compromise the accuracy of the measurement.

The color of 4 in x 4 in x 0.125 injection molded plaques injection molded color plaques was measured according to ASTM E308-06 using illuminant D65 (white light simulating daylight) at 10° angle (1964 CIE).

L*, a* and b* color coordinates were measured using a Gretag Macbeth Color Eye Ci5 Spectrophotometer, with tribeam diffuse/8 "6" sphere optical geometry, a bandpass of 10 nm, a spectral range of 360 nm to 750 nm per CIE Lab standards using illuminant D65 and a 10 degree observer. Thus, the L, a and b color coordinates measured by this test correspond to the lightness scale (L), green-red hue scale (a) and the blue-yellow hue scale (b).

Composition, mechanical properties, color properties and physical properties of the nine compositions are summarized in Table 1.

**Table 1**

| **Examples** | **Comp. example 1 (C1)** | **2** | **3** | **4** | **5** | **6** | **7** | **8** | **9** |
|---|---|---|---|---|---|---|---|---|---|
| **KetaSpire KT-820P PEEK (wt %)** | 100.0 | 99.5 | 98.8 | 97.5 | 95.0 | 92.5 | 90.0 | 95.0 | 90.0 |
| **Boron Nitride, Boronid^{®} S1-SF (wt %)** | - | 0.5 | 1.2 | 2.5 | 5.0 | 7.5 | 10.0 | | |
| **Boron Nitride, Boronid^{®} S15 (wt %)** | | | | | | | | 5.0 | 10.0 |
| **Mechanical properties** | | | | | | | | | |
| Tensile Yield Strength (psi) | 13555 | 13600 | 13700 | 13715 | 13610 | 13630 | 13640 | 13550 | 13600 |
| Tensile Modulus (Ksi) | 536 | 558 | 580 | 611 | 679 | 759 | 839 | 675 | 830 |
| Tensile Yield Elongation (%) | 5.1 | 5.0 | 4.9 | 5.0 | 4.9 | 4.80 | 4.7 | 4.9 | 4.7 |
| Tensile Elongation at Break (%) | 24 | 35 | 31 | 33 | 40 | 46 | 41 | 23 | 23 |
| Flexural Strength (psi) | 20675 | 21000 | 21300 | 21675 | 21320 | 22310 | 22860 | | |
| Flexural Modulus (Ksi) | 532 | 558 | 573 | 601 | 625 | 710 | 775 | | |
| Notched Izod (ft-lb/in) | 1.77 | 1.51 | 1.45 | 1.77 | 2.15 | 2.12 | 2.07 | 1.83 | 1.79 |
| No Notch Izod (ft-lb/in) | NB | NB | NB | NB | NB | NB | NB | NB | NB |
| Dynatup - Total Energy (ft-lb) | 52.0 | 57.7 | 55.6 | 53.5 | 51.5 | 53.7 | 50.6 | 51.0 | 38.4 |
| Dynatup - Max. Load (lb) | 1426 | - | - | 1499 | 1513 | 1547 | 1640 | 1627 | 1478 |
| Dynatup - Energy at Max Load (ft-lb) | 39.0 | - | - | 41.1 | 40.1 | 42.8 | 44.0 | 45.0 | 36.0 |
| Dynatup - Max. Deflection (in) | 0.64 | - | - | 0.64 | 0.62 | 0.64 | 0.62 | 0.64 | 0.56 |
| **Color properties** | | | | | | | | | |
| CIE Lab L* Color Value | 65.2 | 68.9 | 71.1 | 73.0 | 76.1 | 78.3 | 79.6 | 70.5 | 73.3 |
| CIE Lab a* Color Value | 1.76 | 1.74 | 1.40 | 1.38 | 1.27 | 1.11 | 0.98 | 1.82 | 1.52 |
| CIE Lab b* Color Value | 7.07 | 7.45 | 8.58 | 9.95 | 10.76 | 11.02 | 11.15 | 11.13 | 12.09 |
| **Physical properties** | | | | | | | | | |
| DMA Storage Modulus at 200°C (Pa) | 1.30 E8 | - | - | 1.80 E8 | 2.11 E8 | 2.65 E8 | 1.81 E8 | 2.46 E8 | .1.80 E8 |
| HDT [Annealed 200°C/2h] (°C) | 158°C | - | - | 162°C | 163°C | 165°C | 161°C | 167°C | 162°C |

| | | | | | | | | | |
|---|---|---|---|---|---|---|---|---|---|
| NB = No break | | | | | | | | | |

### General description of the compounding process of carbon fiber reinforced PEEK resins

Carbon fiber-reinforced PEEK formulations described in Table 2 were prepared by melt compounding using a twin screw co-rotating intermeshing extruder equipped with 8 barrel sections and an overall L/D ratio of 40. The PEEK powder was tumble blended with either the boron nitride or fed as is (in the case of the Control) in the feed hopper of the extruder. The carbon fiber was fed gravimetrically at the required proportion and was metered at a feed port on barrel section 5 of the extruder. A vacuum vent port at barrel section 7 was used to pull high vacuum on the melt to remove any residual moisture or organic volatiles that may evolve from the sizing of the carbon fiber. The compounded formulations were stranded using a one-hole 3 mm diameter and were cooled on a conveyor belt with a water spray before being fed to a pelletizer to chop the extrudate into pellets. Details of the compounding conditions are shown in Table 3.

**Table 2**

| Properties of carbon fiber reinforced PEEK modified with talc and with boron nitride | | | |
|---|---|---|---|
| **Examples** | **Comparative Example 10 (C10)** | **11** | **Comparative Example 12 (C12)** |
| **KetaSpire KT-820P PEEK** | 70.0 | 68.6 | 68.6 |
| **Boron Nitride, Boronid^{®} S1-SF** | - | 2.0 | - |
| **Talc, Mistron Vapor R** | - | - | 2.0 |
| **Carbon Fiber, SGL C30 APS 006** | 30.0 | 29.4 | 29.4 |
| **Mechanical properties** | | | |
| Tensile Strength (psi) @0.2"/min | 32050 | 31410 | 30920 |
| Tensile Modulus (Ksi) | 3413 | 3470 | 3407 |
| Tensile Elongation at Break (%) | 2.17 | 2.32 | 2.24 |
| Flex Strength (psi) | 50290 | 49310 | 48970 |
| Flex Modulus (Ksi) | 2684 | 2729 | 2689 |
| Flex Strain at Break (%) | 2.58 | 2.61 | 2.56 |
| Notched Izod (ft-lb/in) | 1.76 | 1.63 | 1.55 |
| No Notch Izod (ft-lb/in) | 15.5 | 16.1 | 15.9 |

**Table 3**

| Compounding conditions and process parameters used to make formulations listed in Table 3. | | | |
|---|---|---|---|
| **Examples** | **Comparative Example 10 (C10)** | **11** | **Comparative Example 12 (C12)** |
| *Barrel Zone Temperature (°C)* | | | |
| Barrel Section 1 | No Heat | No Heat | No Heat |
| Barrel Section 2 (Set Point/Actual) | 330/329 | 330/330 | 330/329 |
| Barrel Section 3 (Set Point/Actual) | 330/330 | 330/330 | 330/330 |
| Barrel Section 4 (Set Point/Actual) | 330/331 | 330/330 | 330/331 |
| Barrel Section 5 (Set Point/Actual) | 330/ 334 | 330/ 332 | 330/ 332 |
| Barrel Section 6 (Set Point/Actual) | 340/ 343 | 340/342 | 340/341 |
| Barrel Section 7 (Set Point/Actual) | 340/342 | 340/342 | 340/340 |
| Barrel Section 8 (Set Point/Actual) | 340/340 | 340/342 | 340/341 |
| Adapter (Set Point/Actual) | 340/340 | 340/341 | 340/ 341 |
| Die (Set Point/Actual) | 340/340 | 340/341 | 340/341 |
| | | | |
| Actual Melt Temperature (°C) | 402 | 407 | 423 |
| Screw Speed (rpm) | 230 | 230 | 235 |
| Vent Vacuum on BBL Section 7 (in Hg) | 29 | 29 | 29 |
| Feed Rate, Main (lb/hr) | 7.0 | 7.06 | 7.06 |
| Feed Rate, Carbon Fiber (lb/hr) | 3.0 | 2.94 | 2.94 |
| Feed Rate, Total (lb/hr) | 10.0 | 10.0 | 10.0 |

## Claims

1. A prosthetic device comprising at least one part made of a polymer composition [composition (C), herein after] comprising
(i) at least one polyaryletherketone polymer [(PAEK) polymer],
(ii) at least one nitride (NI) of an element having an electronegativity (ε) of from 1.3 to 2.5, as defined in « Handbook of Chemistry and Physics », CRC Press, 64^{th} edition, pages B-65 to B-158.

2. The prosthetic device according to claim 1, wherein more than 50 % moles of recurring units of (PAEK) polymer are recurring units (R_{PAEK}) selected from those of formulae (J-A) to (J-O), herein below : 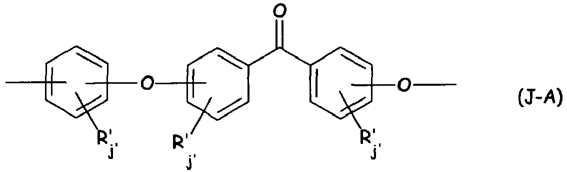 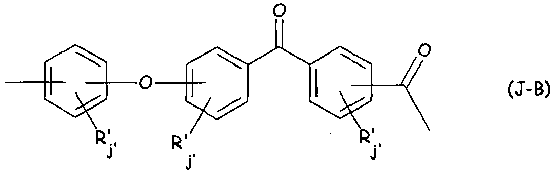 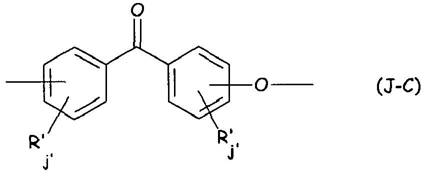 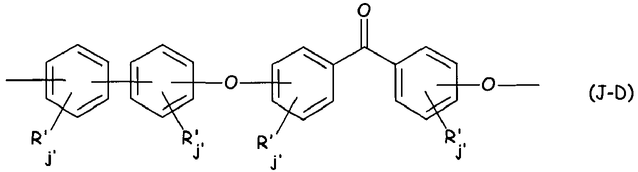 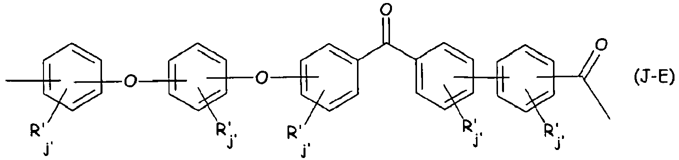 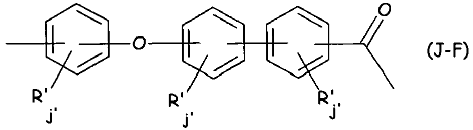 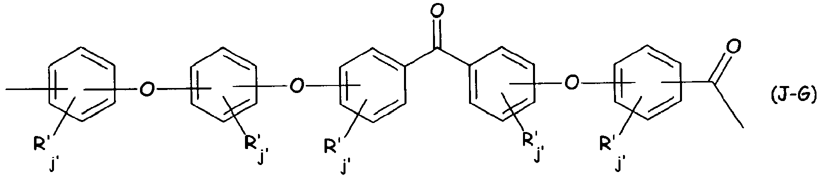 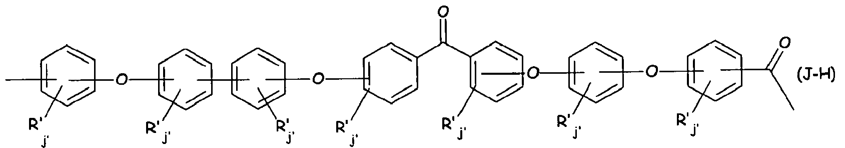 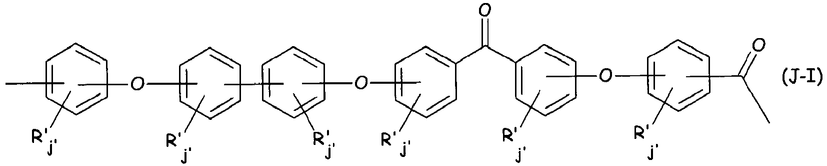 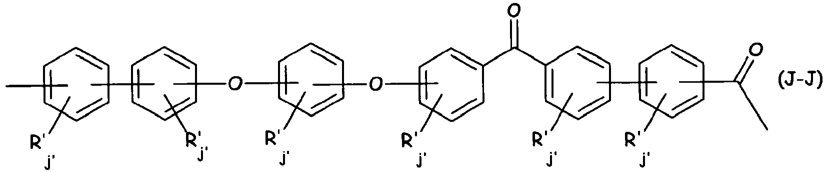 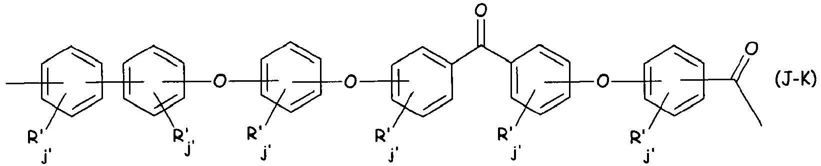 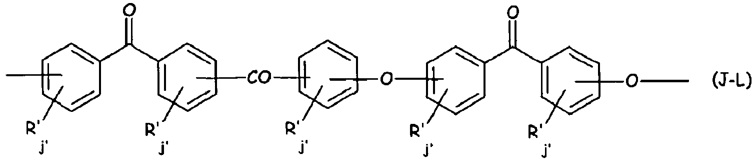 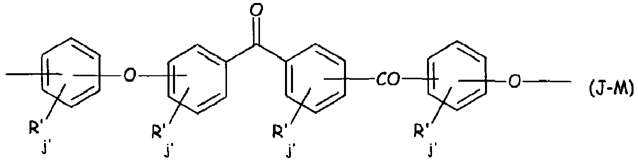 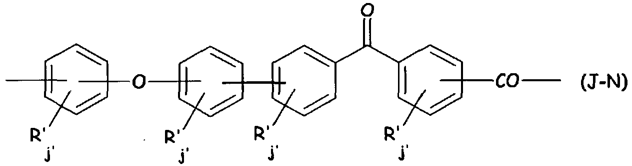 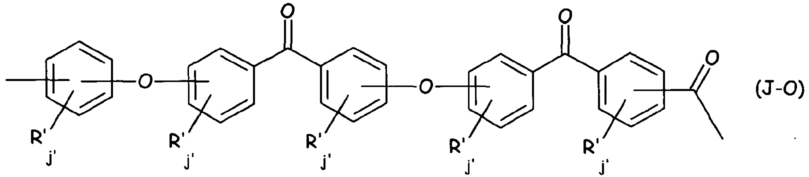 wherein :
- each of R', equal to or different from each other, is selected from the group consisting of halogen, alkyl, alkenyl, alkynyl, aryl, ether, thioether, carboxylic acid, ester, amide, imide, alkali or alkaline earth metal sulfonate, alkyl sulfonate, alkali or alkaline earth metal phosphonate, alkyl phosphonate, amine and quaternary ammonium ;
- j' is zero or is an integer from 0 to 4.

3. The prosthetic device according to any one of claims 1 or 2, wherein more than 50 % moles of recurring units of the (PAEK)polymer are recurring units (R_{PAEK}) selected from those of formulae (J'-A) to (J'-O) herein below :

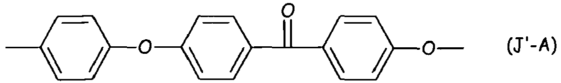

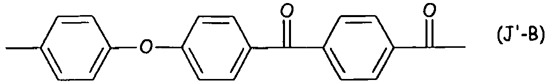

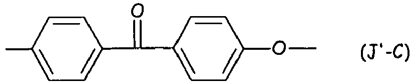

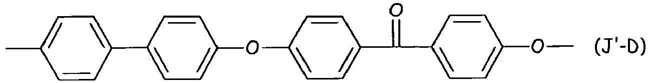

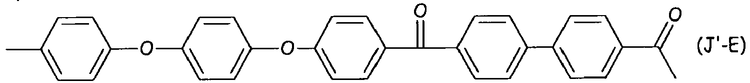

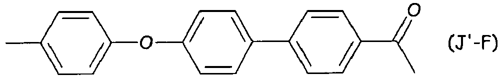

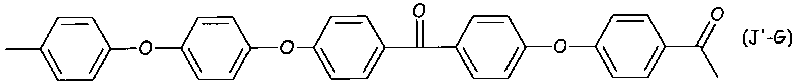

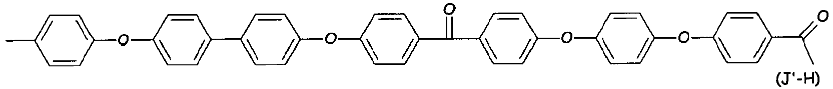

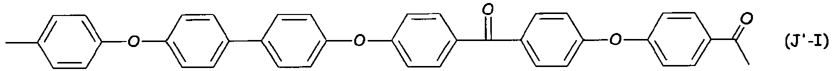

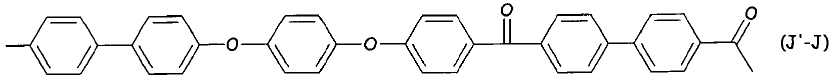

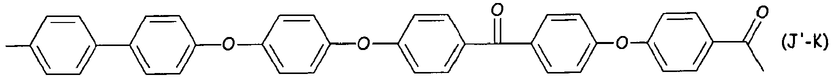

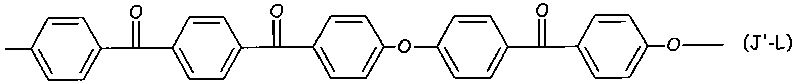

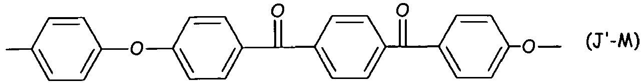

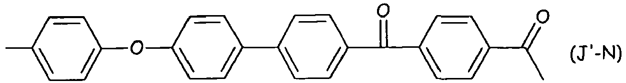

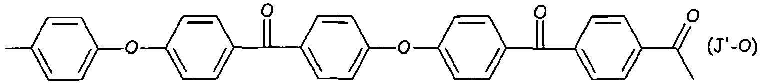

4. The prosthetic device according to any one of claims 1 to 3, wherein the nitride (NI) is a nitride of an element having an electronegativity of at least 1.6.

5. The prosthetic device according to any one of claims 1 to 4, wherein the nitride (NI) is boron nitride.

6. The prosthetic device according to any one of claims 1 to 5, wherein the nitride (NI) is present in an amount of at most 50.0 % wt, based on the total weight of the composition (C).

7. The prosthetic device according to anyone of claims 1 to 6, wherein the polymer composition (C) further comprises at least one other thermoplastic polymer (polymer T), different from the (PAEK) polymer.

8. The prosthetic device according to anyone of claims 1 to 7, wherein the polymer composition (C) further comprises one or more ingredients other than the (PAEK) polymer [ingredient (I)].

9. The prosthetic device according to anyone of claims 1 to 8, wherein the polymer composition (C) further comprises at least one reinforcing filler.

10. The prosthetic device according to claim 9, wherein the reinforcing filler is chosen from wollastonite and glass fiber.

11. The prosthetic device according to claim 9, wherein the reinforcing filler is a carbon fiber.

12. The prosthetic device according to anyone of claims 1 to 11, wherein it is selected from ligaments, tendons, cartilage, bones, hip joints, knee prosthesis and spinal disc orthoprosthesis.

13. Method for the manufacture of the part of the prosthetic device according to anyone of the claims 1 to 12, comprising a step of injection molding and solidification of the polymer composition (C).

14. A part of a prosthetic device according to anyone of the claims 1 to 13.

## Patentansprüche

1. Prothesevorrichtung, umfassend wenigstens einen Teil aus einer Polymerzusammensetzung [nachstehend Zusammensetzung (C)], umfassend
(i) wenigstens ein Polyaryletherketonpolymer [(PAEK)-Polymer],
(ii) wenigstens ein Nitrid (NI) eines Elements mit einer Elektronegativität (ε) von 1,3 bis 2,5, wie definiert im "Handbook of Chemistry and Physics", CRC Press, 64. Auflage, Seiten B-65 bis B-158.

2. Prothesevorrichtung gemäß Anspruch 1, wobei mehr als 50 mol-% der Wiederholungseinheiten des (PAEK)-Polymers Wiederholungseinheiten (R_{PAEK}) ausgewählt aus jenen der nachstehenden Formeln (J-A) bis (J-O) sind : 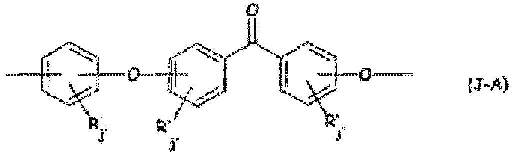 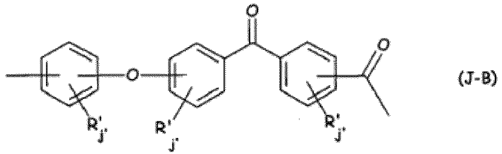 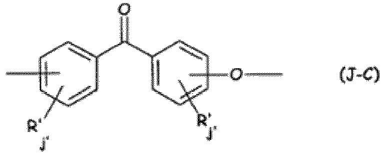 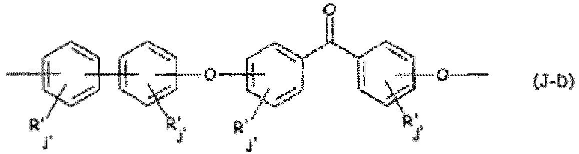 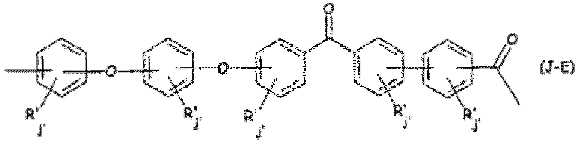 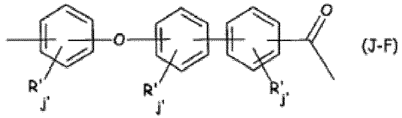 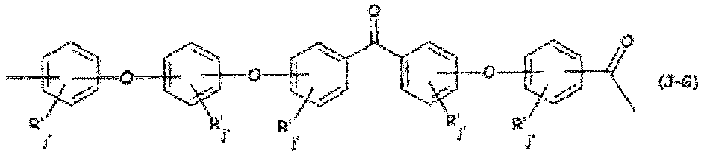 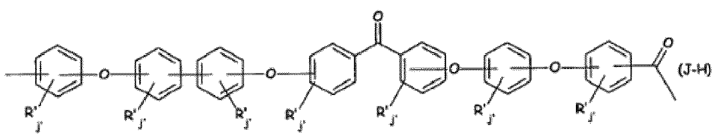 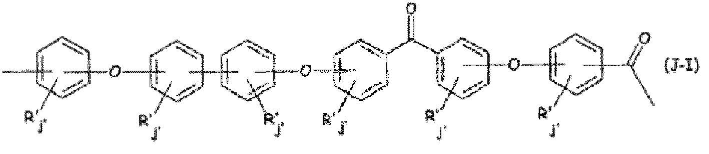 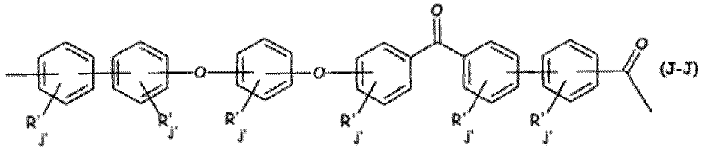 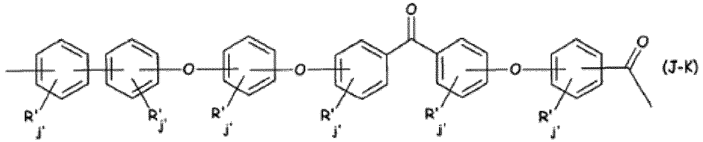 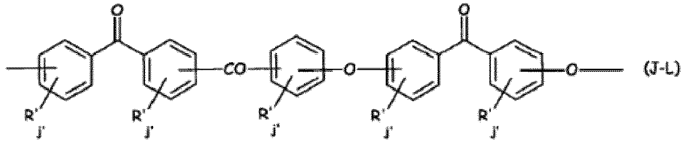 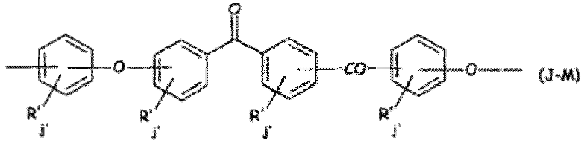 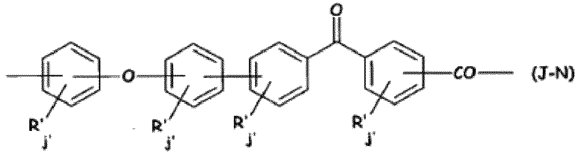 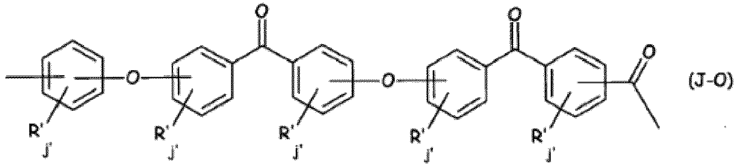 wobei :
- jedes von R', die gleich oder voneinander verschieden sind, ausgewählt ist aus der Gruppe bestehend aus Halogen, Alkyl, Alkenyl, Alkinyl, Aryl, Ether, Thioether, Carbonsäure, Ester, Amid, Imid, Alkali- oder Erdalkalimetallsulfonat, Alkylsulfonat, Alkali- oder Erdalkaliphosphonat, Alkylphosphonat, Amin und quaternärem Ammonium;
- j' null oder eine ganze Zahl von 0 bis 4 ist.

3. Prothesevorrichtung gemäß einem der Ansprüche 1 oder 2, wobei mehr als 50 mol-% der Wiederholungseinheiten des (PAEK)-Polymers Wiederholungseinheiten (R_{PAEK}) ausgewählt aus jenen der nachstehenden Formeln (J'-A) bis (J'-O) sind :

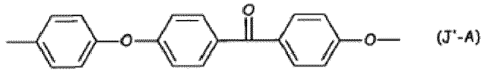

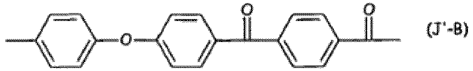

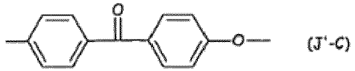

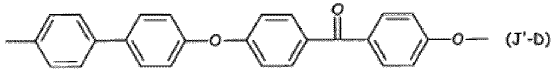

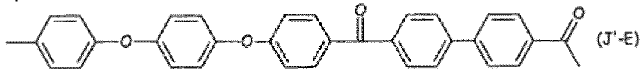

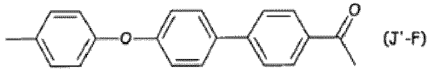

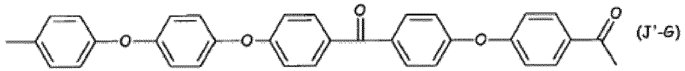

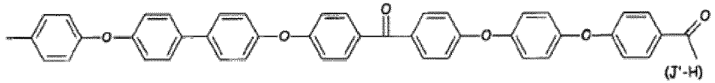

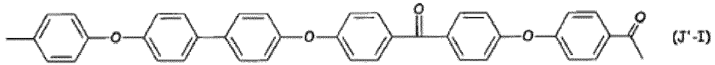

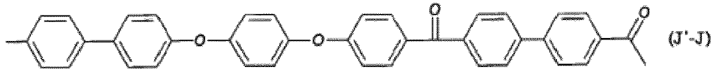

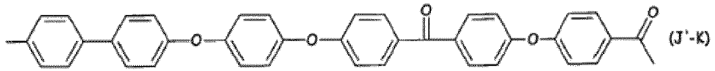

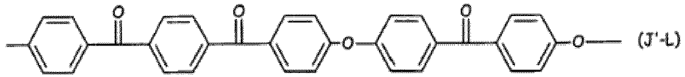

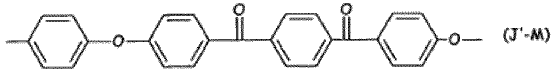

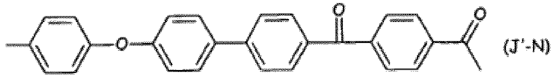

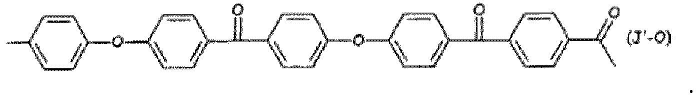

4. Prothesevorrichtung gemäß einem der Ansprüche 1 bis 3, wobei das Nitrid (NI) ein Nitrid eines Elements mit einer Elektronegativität von wenigstens 1,6 ist.

5. Prothesevorrichtung gemäß einem der Ansprüche 1 bis 4, wobei das Nitrid (NI) Bornitrid ist.

6. Prothesevorrichtung gemäß einem der Ansprüche 1 bis 5, wobei das Nitrid (NI) in einer Menge von höchstens 50,0 Gew.-% bezogen auf das Gesamtgewicht der Zusammensetzung (C) vorhanden ist.

7. Prothesevorrichtung gemäß einem der Ansprüche 1 bis 6, wobei die Polymerzusammensetzung (C) ferner wenigstens ein anderes thermoplastisches Polymer (Polymer T), das von dem (PAEK)-Polymer verschieden ist, umfasst.

8. Prothesevorrichtung gemäß einem der Ansprüche 1 bis 7, wobei die Polymerzusammensetzung (C) ferner einen oder mehrere Bestandteile, das von dem (PAEK)-Polymer [Bestandteil (I)] verschieden ist, umfasst.

9. Prothesevorrichtung gemäß einem der Ansprüche 1 bis 8, wobei die Polymerzusammensetzung (C) ferner wenigstens einen Verstärkungsfüllstoff umfasst.

10. Prothesevorrichtung gemäß Anspruch 9, wobei der Verstärkungsfüllstoff ausgewählt ist aus Wollastonit und Glasfaser.

11. Prothesevorrichtung gemäß Anspruch 9, wobei der Verstärkungsfüllstoff eine Karbonfaser ist.

12. Prothesevorrichtung gemäß einem der Ansprüche 1 bis 11, die ausgewählt ist aus Bändern, Sehnen, Knorpel, Knochen, Hüftgelenken, Knieprothesen und Bandscheiben-Orthoprothesen.

13. Verfahren zum Herstellen des Teils der Prothesevorrichtung gemäß einem der Ansprüche 1 bis 12, umfassend einen Schritt des Spritzgießens und Veifestigens der Polymerzusammensetzung (C).

14. Teil einer Prothesevorrichtung gemäß einem der Ansprüche 1 bis 13.

## Revendications

1. Dispositif prothétique comprenant au moins une partie constituée d'une composition de polymère [composition (C) ci-après] comprenant
(i) au moins un polymère de polyaryléthercétone [polymère (PAEK)],
(ii) au moins un nitrure (NI) d'un élément présentant une électronégativité (ε) comprise entre 1,3 et 2,5, comme défini dans le « Handbook of Chemistry and Physics », CRC Press, 64^{e} édition, pages B-65 à B-158.

2. Dispositif prothétique selon la revendication 1, où plus de 50 % molaires des motifs de répétition du polymère (PAEK) sont des motifs de répétition (R_{PAEK}) choisis parmi ceux de formule (J-A) à (J-O), ci-après : 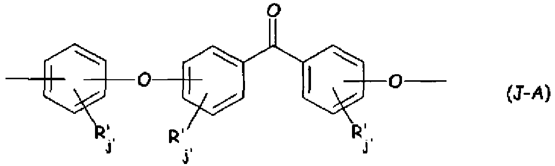 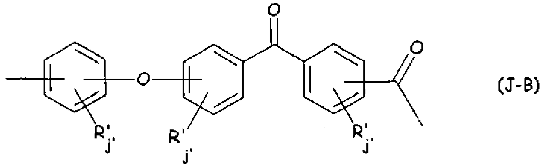 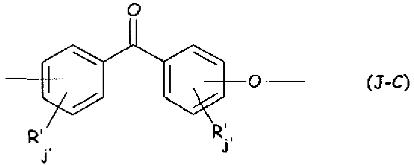 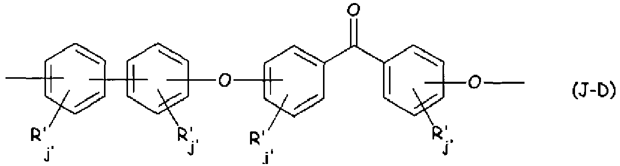 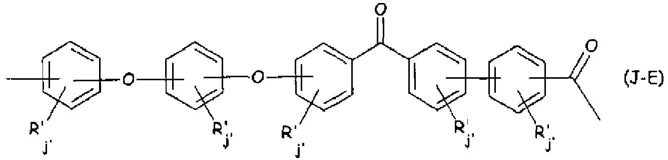 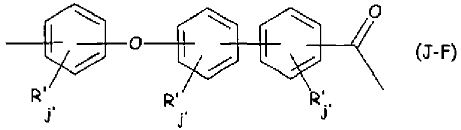 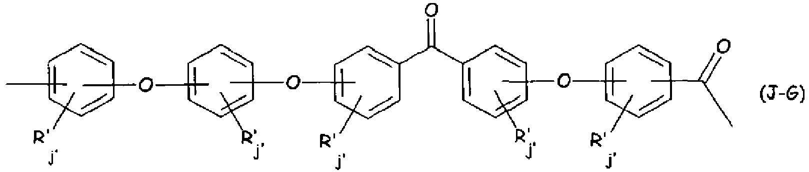 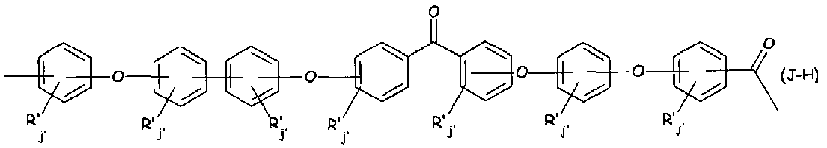 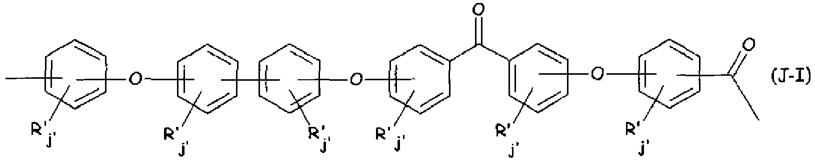 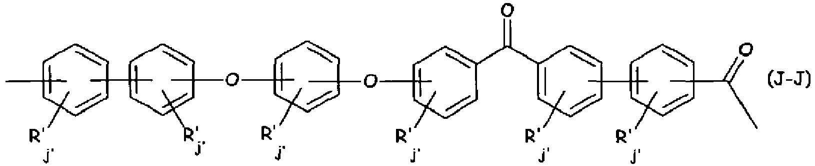 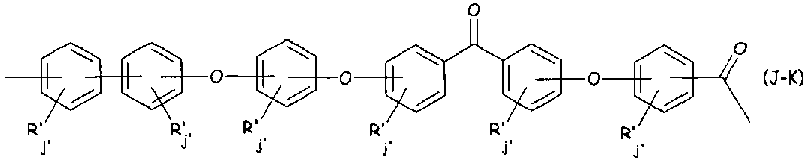 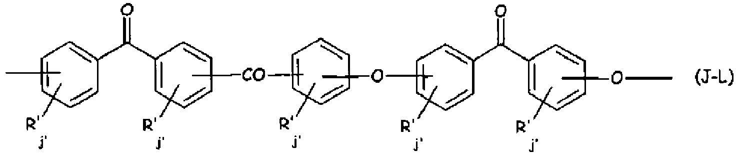 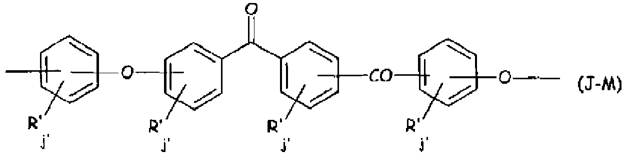 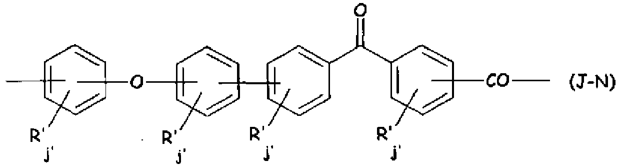 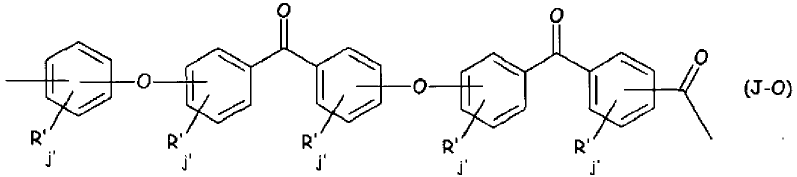 où :
- chacun des radicaux R', identiques ou différents les uns des autres, est choisi dans le groupe constitué par les atomes d'halogène et les groupements alkyle, alcényle, alcynyle, aryle, éther, thioéther, acide carboxylique, ester, amide, imide, sulfonate de métal alcalin ou alcaline-terreux, alkylsulfonate, phosphonate de métal alcalin ou alcalino-terreux, alkylphosphonate, amine et ammonium quaternaire ;
- j' est égal à zéro ou représente un entier compris entre 0 et 4.

3. Dispositif prothétique selon l'une quelconque des revendications 1 ou 2, où plus de 50 % molaires des motifs de répétition du polymère (PAEK) sont des motifs de répétition (R_{PAEK}) choisis parmi ceux de formule (J'-A) à (J'-O), ci-après :

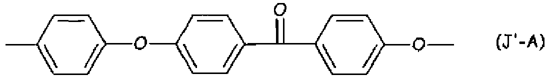

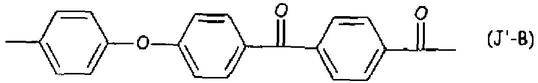

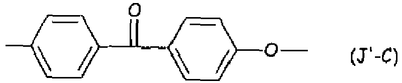

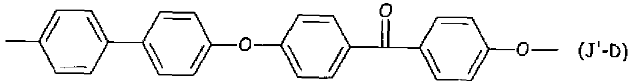

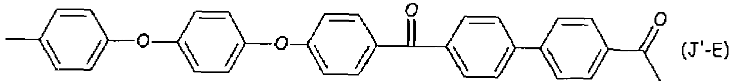

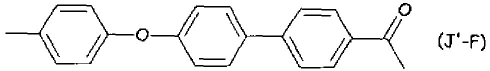

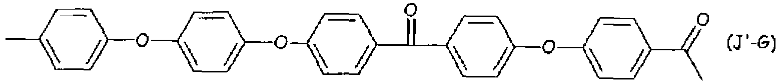

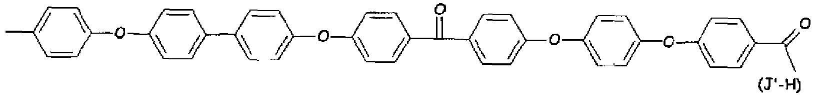

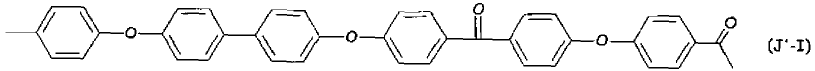

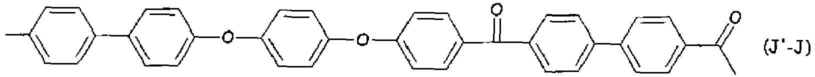

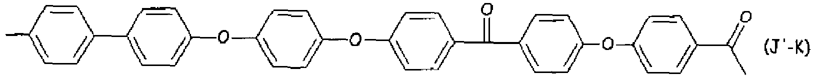

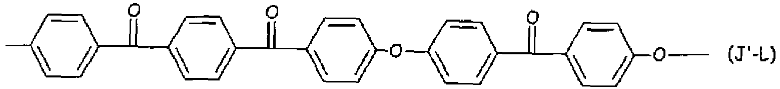

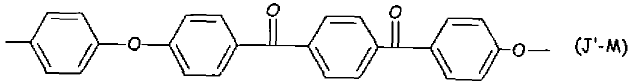

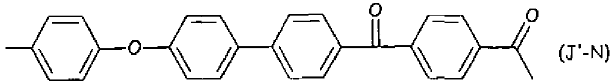

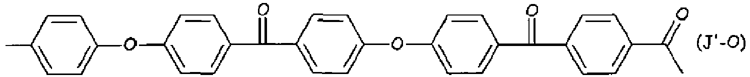

4. Dispositif prothétique selon l'une quelconque des revendications 1 à 3, où le nitrure (NI) représente un nitrure d'un élément présentant une électronégativité d'au moins 1,6.

5. Dispositif prothétique selon l'une quelconque des revendications 1 à 4, où le nitrure (NI) est le nitrure de bore.

6. Dispositif prothétique selon l'une quelconque des revendications 1 à 5, où le nitrure (NI) est présent à une teneur d'au plus 50,0 % en masse, par rapport à la masse totale de la composition (C).

7. Dispositif prothétique selon l'une quelconque des revendications 1 à 6, où la composition de polymère (C) comprend en outre au moins un autre polymère thermoplastique (polymère T) différent du polymère (PAEK).

8. Dispositif prothétique selon l'une quelconque des revendications 1 à 7, où la composition de polymère (C) comprend en outre un ou plusieurs composants différents du polymère (PAEK) [composant (I)].

9. Dispositif prothétique selon l'une quelconque des revendications 1 à 8, où la composition de polymère (C) comprend en outre au moins une charge de renfort.

10. Dispositif prothétique selon la revendication 9, où la charge de renfort est choisie parmi la wollastonite et la fibre de verre.

11. Dispositif prothétique selon la revendication 9, où la charge de renfort est une fibre de carbone.

12. Dispositif prothétique selon l'une quelconque des revendications 1 à 11, où celui-ci est choisi parmi les ligaments, tendons, cartilages, os, articulations de hanche, prothèse de genou et orthoprothèse de disque vertébral.

13. Procédé de fabrication de la partie du dispositif prothétique selon l'une quelconque des revendications 1 à 12, comprenant une étape de moulage par injection et de solidification de la composition de polymère (C).

14. Partie d'un dispositif prothétique selon l'une quelconque des revendications 1 à 13.
